# EUROPEAN PATENT APPLICATION

(11) **EP 4 053 144 A2**
(43) Date of publication of application: **07.09.2022**
(21) Application number: 22160196.6
(22) Date of filing: 04.03.2022
(51) Int. Cl.: C07K 7/06, A01N 37/46

(54) **PEPTIDES WITH PLANT PROTECTION ACTION**

(30) Priority: 04.03.2021 IT 202100005057
(71) Applicant: Università degli Studi di Padova, 35122 Padova (IT)
(72) Inventor: Marta, DE ZOTTI, PADOVA (IT); Francesco, FAVARON, VEGGIANO (PD) (IT); Luca, SELLA, LEGNARO (PD) (IT); Silvio, TUNDO, FORMIA (LT) (IT)
(74) Representative: Marchioro, Paolo

(57) **Abstract**

Use of a peptide or of a salt thereof as a plant protection product against pathogenic micro-organisms in plants. The peptide has the following general formula (I): 1-octanoyl-X-Aib-Y-Z (I), wherein X is selected from the group consisting of Aib-Lys(HCl)-Leu, Aib-Gly-Leu, Leu, or X is absent; Y is selected from the group consisting of Lys(HCl), Lys(HCl)-Gly-Leu-Aib-Lys(HCl), Lys(HCl)-Lys(HCl)-Leu-Aib-Lys(HCl), Gly-Gly-Leu-Aib-Lys(HCI) and Lys(HCl)-Lys(HCI)-Leu-Aib-Gly; Z is selected from the group consisting of Lol, Ilol, Ile-NH₂, Leu-NH₂, Ile-Lol and Ile-Leu-NH₂; and wherein if, at the same time, X is Aib-Gly-Leu or is absent and Y is Lys(HCl)-Lys(HCl)-Leu-Aib-Gly, then Z is not Ile-Lol and is not Ile-Leu-NH₂.

SEQUENCE LISTING

<110> Università degli studi di Padova

<120> PEPTIDI AD AZIONE FITOSANITARIA

<130> 55.436UNIPD

<150> IT102021000005057
<151> 2021-03-04

<160> 16

<170> BiSSAP 1.3.6

<210> 1
<211> 11
<212> PRT
<213> Artificial Sequence

<220>
<221> SITE
<222> 1
<223> 1 ottanoile Aib

<220>
<223> Designed peptide K259G6

<220>
<221> SITE
<222> 2
<223> Lys(HCl)

<220>
<221> SITE
<222> 4
<223> Aib

<220>
<221> SITE
<222> 5
<223> Lys(HCl)

<220>
<221> SITE
<222> 8
<223> Aib

<220>
<221> SITE
<222> 9
<223> Lys(HCl)

<220>
<221> SITE
<222> 11
<223> Lol

<400> 1
Xaa Xaa Leu Xaa Xaa Gly Leu Xaa Xaa Ile Xaa
1               5                   10

<210> 2
<211> 11
<212> PRT
<213> Artificial Sequence

<220>
<221> SITE
<222> 1
<223> 1 ottanoile Aib

<220>
<223> Designed peptide K259G6-NH2

<220>
<221> SITE
<222> 2
<223> Lys(HCl)

<220>
<221> SITE
<222> 4
<223> Aib

<220>
<221> SITE
<222> 5
<223> Lys(HCl)

<220>
<221> SITE
<222> 8
<223> Aib

<220>
<221> SITE
<222> 9
<223> Lys(HCl)

<400> 2
Xaa Xaa Leu Xaa Xaa Gly Leu Xaa Xaa Ile Leu
1               5                   10

<210> 3
<211> 11
<212> PRT
<213> Artificial Sequence

<220>
<221> SITE
<222> 1
<223> 1 ottanoile Aib

<220>
<223> Designed peptide K2569

<220>
<221> SITE
<222> 2
<223> Lys(HCl)

<220>

<221> SITE
<222> 4
<223> Aib

<220>
<221> SITE
<222> 5
<223> Lys(HCl)

<220>
<221> SITE
<222> 6
<223> Lys(HCl)

<220>
<221> SITE
<222> 8
<223> Aib

<220>
<221> SITE
<222> 9
<223> Lys(HCl)

<220>
<221> SITE
<222> 11
<223> Lol

<400> 3
Xaa Xaa Leu Xaa Xaa Xaa Leu Xaa Xaa Ile Xaa
1               5                   10

<210> 4
<211> 11
<212> PRT
<213> Artificial Sequence

<220>
<221> SITE
<222> 1
<223> 1 ottanoile Aib

<220>
<223> Designed peptide K2569-NH2

<220>
<221> SITE
<222> 2
<223> Lys(HCl)

<220>
<221> SITE
<222> 4
<223> Aib

<220>
<221> SITE
<222> 5
<223> Lys(HCl)

<220>

<221> SITE
<222> 6
<223> Lys(HCl)

<220>
<221> SITE
<222> 8
<223> Aib

<220>
<221> SITE
<222> 9
<223> Lys(HCl)

<400> 4
Xaa Xaa Leu Xaa Xaa Xaa Leu Xaa Xaa Ile Leu
1               5                   10

<210> 5
<211> 11
<212> PRT
<213> Artificial Sequence


<220>
<221> SITE
<222> 1
<223> 1 ottanoile Aib

<220>
<223> Designed peptide K9

<220>
<221> SITE
<222> 4
<223> Aib

<220>
<221> SITE
<222> 8
<223> Aib

<220>
<221> SITE
<222> 9
<223> Lys(HCl)

<220>
<221> SITE
<222> 11
<223> Lol

<400> 5
Xaa Gly Leu Xaa Gly Gly Leu Xaa Xaa Ile Xaa
1               5                   10

<210> 6
<211> 11
<212> PRT
<213> Artificial Sequence


<220>

<221> SITE
<222> 1
<223> 1 ottanoile Aib

<220>
<223> Designed peptide K9-NH2

<220>
<221> SITE
<222> 4
<223> Aib

<220>
<221> SITE
<222> 8
<223> Aib

<220>
<221> SITE
<222> 9
<223> Lys(HCl)

<400> 6
Xaa Gly Leu Xaa Gly Gly Leu Xaa Xaa Ile Leu
1               5                   10

<210> 7
<211> 11
<212> PRT
<213> Artificial Sequence


<220>
<221> SITE
<222> 1
<223> 1 ottanoile Aib

<220>
<223> Designed peptide K256

<220>
<221> SITE
<222> 2
<223> Lys(HCl)

<220>
<221> SITE
<222> 4
<223> Aib

<220>
<221> SITE
<222> 5
<223> Lys(HCl)

<220>
<221> SITE
<222> 6
<223> Lys(HCl)

<220>
<221> SITE
<222> 8

<223> Aib

<220>
<221> SITE
<222> 11
<223> Lol

<400> 7
Xaa Xaa Leu Xaa Xaa Xaa Leu Xaa Gly Ile Xaa
1               5                   10

<210> 8
<211> 11
<212> PRT
<213> Artificial Sequence



<220>
<221> SITE
<222> 1
<223> 1 ottanoile Aib

<220>
<223> Designed peptide K256-NH2

<220>
<221> SITE
<222> 2
<223> Lys(HCl)

<220>
<221> SITE
<222> 4
<223> Aib

<220>
<221> SITE
<222> 5
<223> Lys(HCl)

<220>
<221> SITE
<222> 6
<223> Lys(HCl)

<220>
<221> SITE
<222> 8
<223> Aib

<400> 8
Xaa Xaa Leu Xaa Xaa Xaa Leu Xaa Gly Ile Leu
1               5                   10

<210> 9
<211> 5
<212> PRT
<213> Artificial Sequence



<220>
<221> SITE
<222> 1

<223> 1 ottanoile Leu

<220>
<223> Designed peptide

<220>
<221> SITE
<222> 2
<223> Aib

<220>
<221> SITE
<222> 3
<223> Lys(HCl)

<220>
<221> SITE
<222> 5
<223> Lol

<400> 9
Xaa Xaa Xaa Ile Xaa
1               5

<210> 10
<211> 5
<212> PRT
<213> Artificial Sequence


<220>
<221> SITE
<222> 1
<223> 1 ottanoile Leu

<220>
<223> Designed peptide

<220>
<221> SITE
<222> 2
<223> Aib

<220>
<221> SITE
<222> 3
<223> Lys(HCl)

<400> 10
Xaa Xaa Xaa Ile Leu
1               5

<210> 11
<211> 4
<212> PRT
<213> Artificial Sequence


<220>
<221> SITE
<222> 1
<223> 1 ottanoile Leu

<220>
<223> Designed peptide

<220>
<221> SITE
<222> 2
<223> Aib

<220>
<221> SITE
<222> 3
<223> Lys(HCl)

<220>
<221> SITE
<222> 4
<223> Lol

<400> 11
Xaa Xaa Xaa Xaa
1

<210> 12
<211> 4
<212> PRT
<213> Artificial Sequence


<220>
<221> SITE
<222> 1
<223> 1 ottanoile Leu

<220>
<223> Designed peptide

<220>
<221> SITE
<222> 2
<223> Aib

<220>
<221> SITE
<222> 3
<223> Lys(HCl)

<400> 12
Xaa Xaa Xaa Leu
1

<210> 13
<211> 4
<212> PRT
<213> Artificial Sequence


<220>
<221> SITE
<222> 1
<223> 1 ottanoile Leu

<220>
<223> Designed peptide

<220>
<221> SITE
<222> 2
<223> Aib

<220>
<221> SITE
<222> 3
<223> Lys(HCl)

<220>
<221> SITE
<222> 4
<223> Ilol

<400> 13
Xaa Xaa Xaa Xaa
1

<210> 14
<211> 4
<212> PRT
<213> Artificial Sequence

<220>
<221> SITE
<222> 1
<223> 1 ottanoile Leu

<220>
<223> Designed peptide

<220>
<221> SITE
<222> 2
<223> Aib

<220>
<221> SITE
<222> 3
<223> Lys(HCl)

<400> 14
Xaa Xaa Xaa Ile
1

<210> 15
<211> 4
<212> PRT
<213> Artificial Sequence

<220>
<221> SITE
<222> 1
<223> 1 ottanoile Aib

<220>
<223> Designed peptide

<220>

<221> SITE
<222> 2
<223> Lys(HCl)

<220>
<221> SITE
<222> 4
<223> Lol

<400> 15
Xaa Xaa Ile Xaa
1

<210> 16
<211> 4
<212> PRT
<213> Artificial Sequence

<220>
<221> SITE
<222> 1
<223> 1 ottanoile Aib

<220>
<223> Designed peptide

<220>
<221> SITE
<222> 2
<223> Lys(HCl)

<400> 16
Xaa Xaa Ile Leu
1

## Description

The present invention relates to novel analogue peptides of the natural peptaboil trichogin GA IV and their use as plant protection products for the treatment and/or prevention of diseases caused by pathogenic micro-organisms to the plants.

The present invention also relates to a plant protection composition comprising at least one of such peptides and its use as a plant protection product against pathogenic micro-organisms of the plants.

### Background

The present invention relates to the field of fighting biological pathogen agents of plant crops, in particular biological agents such as fungi, oomycetes and/or phytopathogenic bacteria.

In order to protect and prevent the damaging effects of biological agents on plants, numerous types of agrochemicals and biocides are in use.

The term agrochemical refers to a chemical compound, natural or synthetic, used in agriculture to fight plant pests and diseases, to protect crops from harmful agents, and to improve their productivity.

The term biocide, on the other hand, refers to:
- any substance or mixture in the form in which it is supplied to the user, consisting of, containing or capable of generating one or more active ingredients, for the purpose of destroying, eliminating and rendering harmless, preventing the action of or exerting any other control effect on any harmful organism, by any means other than mere physical or mechanical action;
- any substance or mixture, generated from substances or mixtures not covered as such by the first indent, used with the intention of destroying, eliminating, rendering harmless, preventing the action of or exerting any other control effect on any harmful organism, by any means other than mere physical or mechanical action (Article 3 of Regulation (EU) No. 528/2012).

Although the use of biocides and agrochemicals, especially synthetic ones, is aimed at ensuring human well-being and the conservation/development of many plant species, the chemicals contained therein can have harmful effects on human health, plant and animal organisms and, last but not least, on the environment. Their release into the environment, for example, can lead to accumulation phenomena in surface and groundwaters, soil and air.

The active ingredients contained in the agrochemicals are therefore subject to a strict evaluation regime and included in a list of substances permitted throughout the European Union.

The agrochemicals, moreover, can lose efficacy over time due to the emergence of resistant strains.

Therefore, those eco-sustainable agricultural models that offer productive solutions with a reduced environmental impact and increased healthiness of the products themselves are increasingly encouraged.

The improvement interventions that can be carried out in the development of new formulations of agrochemicals and biocides, in order to make them efficient and safe, are referable to the three macro-areas described below:
- improving the safety of operators by eliminating or reducing the use of substances harmful to human health;
- improving consumer safety following the ingestion of agricultural products contaminated by biocides and agrochemicals;
- reducing environmental pollution.

The use of analogue peptides of the natural peptaboil trichogin GA IV and their use as plant protection products are part of this improvement perspective.

As known from document WO2020003220 on behalf of the applicant, peptaboils are secondary metabolites produced by fungi belonging to the genus Trichoderma. These peptaboils are known for their ability to protect plants from pest attacks as they possess antimicrobial activity; some peptaboils act as plant defence stimulants and induce volatile compounds in plants that attract natural enemies of herbivorous insects *(*Vos CMF et al, Mol Plant Pathol, 2015*).*

Peptaibols are peptides produced by fungi in a non-ribosomal manner. They are peptides rich in Aib (alpha-aminoisobutyric acid) amino acid residues and have an aminoalcohol as the C-terminus group. There is also an acyl group at the N-terminus end (a 1-octanoyl group in the case of the peptaibol trichogin GA IV).

Eight peptide analogues of peptaboils having plant protection activity have been described in the mentioned document.

These peptides are advantageous in that they can be used in purified form, are water-soluble and are stable to solar irradiation.

However, these known peptides have some drawbacks.

In particular, such peptides show limited efficacy against certain pathogenic micro-organisms of cereal crops, such as *Pyricularia oryzae.*

Still disadvantageously, such peptides are not particularly active in fighting infections caused on plants by Gram-negative phytopathogenic bacteria. Moreover, inconveniently, such peptides are particularly expensive to produce due to the complexity of their sequence.

### Description of the invention

The object of the present invention is therefore to provide new peptides derived from peptaibols to be used as plant protection products.

In particular, an object of the present invention is that such peptides are suitable for use in fighting pathogenic micro-organisms of the plants, in particular against phytopathogenic bacteria.

Furthermore, an object of the present invention is that such peptides are alternative to those known, in particular are alternative to those described in document WO2020003220, and exhibit a higher plant protection activity than the latter and overcome the known drawbacks thereof.

Again, an object of the present invention is that such peptides can be easily manipulated and used by operators, thus limiting or completely eliminating health risks for agricultural operators and/or end users of plants treated with such peptides.

Furthermore, an object of the present invention is that such peptides exhibit a wide range of efficacy.

Further, an object of the present invention is that such peptides, after carrying out their plant protection activity, are adapted to be degraded into non-toxic amino acids, thus limiting the environmental impact of their use on plants, in particular on agricultural crops.

Again, an object of the present invention is that such peptides are stable under extreme temperature, irradiation and pH conditions, so as to be suitable for use in the field, regardless of the atmospheric and environmental conditions. Last but not least, an object of the present invention is that such peptides are easily producible, so as to reduce their production cost.

The aforesaid objects are achieved by the use of a peptide or of a salt thereof as a plant protection product against pathogenic micro-organisms of the plants, as described in the main claim.

In particular, the present invention relates to the use of a peptide or of a salt thereof as a plant protection product against pathogenic micro-organisms of the plants, wherein such a peptide has general formula (I)

1-octanoyl-X-Aib-Y-Z (I),

wherein X is selected from the group consisting of Aib-Lys(HCI)-Leu, Aib-Gly-Leu, Leu, or X is absent;
Y is selected from the group consisting of Lys(HCI), Lys(HCl)-Gly-Leu-Aib-Lys(HCI), Lys(HCl)-Lys(HCl)-Leu-Aib-Lys(HCl), Gly-Gly-Leu-Aib-Lys(HCI) and Lys(HCl)-Lys(HCl)-Leu-Aib-Gly;
Z is selected from the group consisting of Lol, Ilol, Ile-NH₂, Leu-NH₂, Ile-Lol and Ile-Leu-NH₂;
and wherein if, at the same time, X is Aib-Gly-Leu or is absent and Y is Lys(HCI)-Lys(HCI)-Leu-Aib-Gly, then Z is not Ile-Lol and is not Ile-Leu-NH₂. The objects are further achieved by a peptide as set forth in claim 14.

Furthermore, the objects are also achieved by a plant protection composition and the use of said plant protection composition as a plant protection product against pathogenic micro-organisms of the plants, as set forth in claims 11 and 13, respectively.

Further characteristics of the peptide, the use of the peptide, the plant protection composition and the use of the plant protection composition are set forth in the dependent claims.

In addition, other advantages and features of the peptide, the use of the peptide, the plant protection composition and the use of the plant protection composition will be apparent to a person skilled in the art from the following description of some preferred embodiments of the invention which are given by way of indication but not of limitation.

### Brief description of the figures

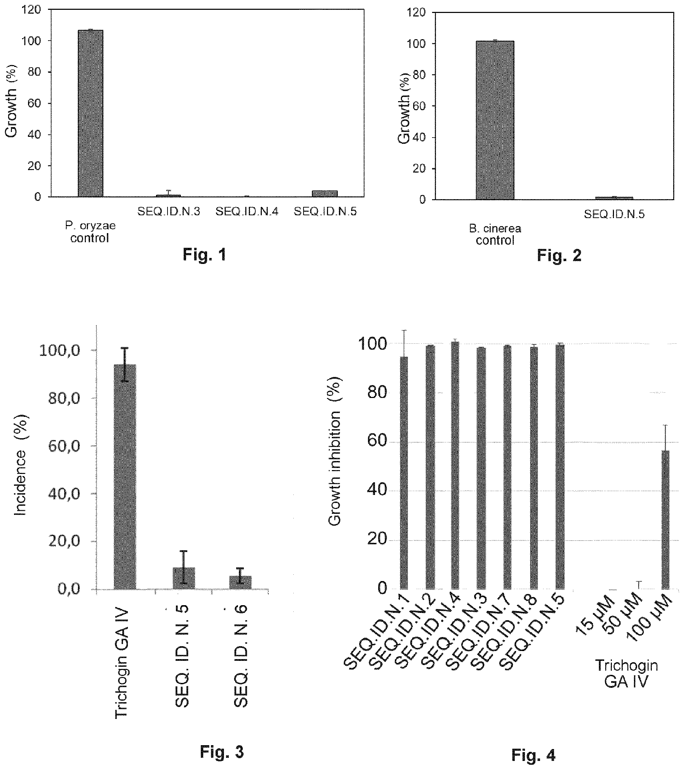

- Figure 1 reports in the graph the growth of the phytopathogenic fungus *Pyricularia oryzae,* inhibited with the *in vitro* use of the peptides of the invention having SEQ. ID. Nos. 3, 4 and 5 at a concentration of 50 µM. The histogram represents the percentage of absorbance values (± standard error) of the wells containing *Pyricularia oryzae* after 96 hours of incubation with the peptide compared to the maximum absorbance measured in the control wells containing the fungus but not inoculated with the peptide, obtained by performing at least three independent biological replicates. The use of the peptides decreases the growth of the micro-organism by at least 95% compared to the untreated control.
- Figure 2 shows in the graph the growth of the phytopathogenic fungus *Botrytis cinerea,* inhibited with the *in vitro* use of the peptide of the invention having SEQ. ID. No. 5 at a concentration of 15 µM. The histogram represents the percentage of absorbance values (± standard error) of the wells containing *Botrytis cinerea* after 96 hours of incubation with the peptide compared to the maximum absorbance measured in the control wells containing the fungus but not inoculated with the peptide, obtained by performing at least three independent biological replicates. The use of the peptide decreases the growth of the micro-organism by at least 95% compared to the untreated control.
- Figure 3 shows in the graph the percentage of incidence of the infection caused by *Plasmopara viticola* on vine leaf discs, obtained by using the peptides of the invention having SEQ. ID. Nos. 5 and 6 at a concentration of 50 µM. The histogram represents the average incidence rate (± standard error) of *Plasmopara viticola* infections 12 days after inoculation. The incidence is calculated as the ratio of the number of sporulating discs to the total number of treated discs and obtained by performing at least three independent biological replicates. The use of the peptides significantly decreases the *P. viticola* infection.
- Figure 4 reports in the graph the *in vitro* inhibition of the growth of the phytopathogenic bacterium *Xanthomonas campestris pv. campestris*, obtained by using the peptide of the invention having SEQ. ID. Nos. 1, 2, 3, 4, 5, 7 and 8 at a concentration of 15 µM. The histogram represents the average percentage of growth inhibition (± standard error) of *Xanthomonas campestris pv. campestris* determined after 48 h incubation with the peptides, by measuring the reduction in turbidity (Absorbance at 600 nm) of the wells containing the peptides compared to the wells without peptides, and obtained by performing at least three independent replicates. The use of the peptides decreases the growth of the micro-organism by at least 95% compared to the untreated control.

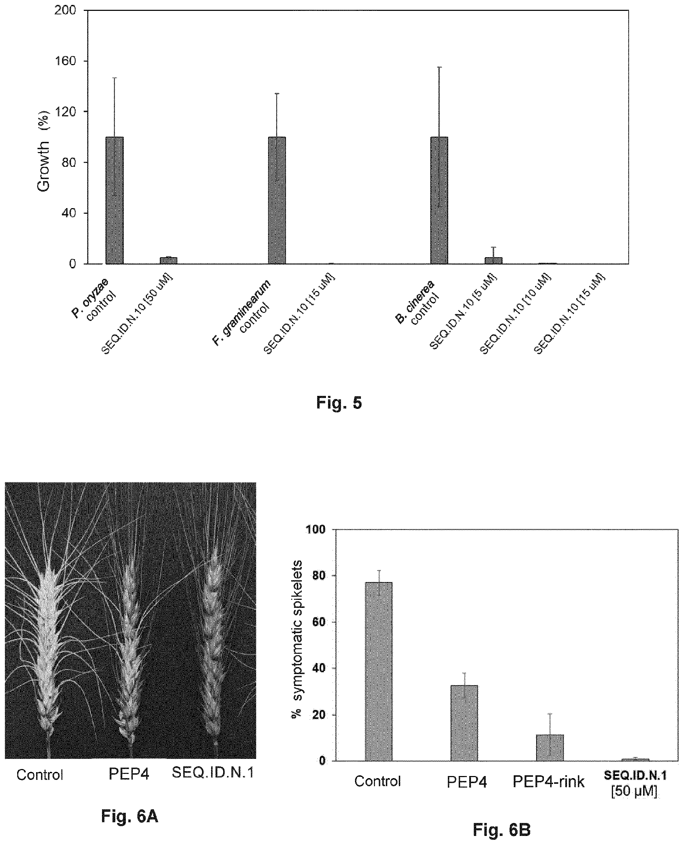

- Figure 5 represents in the graph the growth of the phytopathogenic fungi *Pyricularia oryzae*, *Fusarium graminearum* and *Botrytis cinerea* inhibited with the in *vitro* use of the peptide of the invention having SEQ. ID. No. 10, at a concentration of 50 µM, 15 µM and 5, 10, 15 µM, respectively. The histogram represents the percentage of absorbance values (± standard error) of the wells containing *Pyricularia oryzae, Fusarium graminearum* and *Botrytis cinerea* after 96 hours of incubation with the peptide compared to the maximum absorbance measured in the control wells containing the fungi but not inoculated with the peptide, obtained by performing at least three independent replicates. The use of the peptide decreased the growth of the fungi by at least 95% compared to the untreated control at both concentrations tested.
- Figure 6A shows: an ear of wheat treated with the peptide of the invention having SEQ. ID. No. 1 at a concentration of 50 µM and inoculated with *Fusarium graminearum* (right ear), an ear of wheat treated with the peptide described in WO2020003220 having sequence 1-octanoyl-Aib-Gly-Leu-Aib-Lys(HCl)-Lys(HCl)-Leu-Aib-Gly-Ile-Lol ("PEP4") at a concentration of 50 µM and inoculated with *Fusarium graminearum* (middle ear) and a control ear of wheat not treated with any peptide and inoculated with *Fusarium graminearum* (left ear). The peptide of the invention protects the ear of wheat against infection caused by *Fusarium graminearum,* and furthermore this protection is greater than when using the peptide known as "PEP4".
- Figure 6B shows in the graph the percentage of infected spikelets of wheat after inoculation with 5×10⁵ spores mL⁻¹ of *F. graminearum* and treatment with a solution containing 50 µM of peptide of the invention having SEQ. ID. No. 1 and, for comparison, of the peptide known in WO2020003220 having sequence 1-octanoyl-Aib-Gly-Leu-Aib-Lys(HCl)-Lys(HCl)-Leu-Aib-Gly-Ile-Lol ("PEP4") and of the peptide known in WO2020003220 having sequence 1-octanoyl-Aib-Gly-Leu-Aib-Lys(HCl)-Lys(HCl)-Leu-Aib-Gly-Ile-Leu-NH₂ ("PEP4-rink"). Ears sprayed with water and fungal spores are used as a positive control. The histogram represents the average percentage (± standard error) of symptomatic spikelets determined by counting the number of visually symptomatic spikelets with respect to the total number of spikelets of the respective ear and obtained by performing at least three independent biological replicates. The peptide of the invention decreases infection in treated ears by at least 95% compared to untreated control ears and, in addition, it is also shown to be more effective than the peptides "PEP4" and "PEP4-rink", decreasing the infection by at least 10-30% more than the treatment with the aforesaid known peptides.

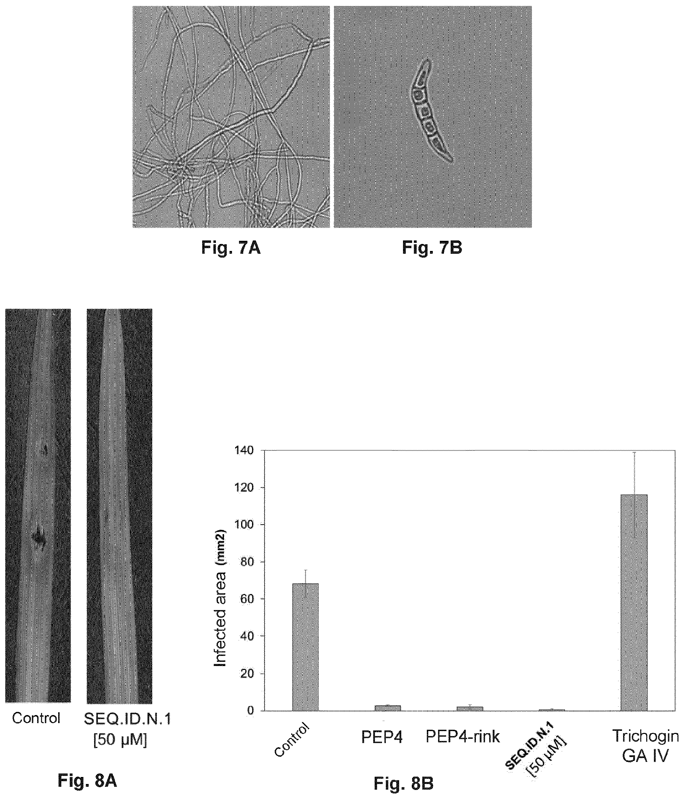

- Figures 7A and 7B show two exemplary images obtained by optical microscope of *Fusarium graminearum* 48 hours after treatment with the peptide of the invention having SEQ. ID. No. 1 (Figure 7B) at 15 µM. It can be noted that germination is inhibited and the cytoplasm is agglutinated. Figure 7A shows the image of the untreated control in which it can be noted that the fungal mycelium germinated normally.
- Figure 8A shows a barley leaf treated with the peptide of the invention having SEQ. ID. No. 1 at a concentration of 50 µM and inoculated with *Pyricularia oryzae* and a control leaf not treated with the peptide and inoculated with the fungus.
- Figure 8B represents in the graph the average area (± standard error) of the lesion produced by *Pyricularia oryzae* in water-treated barley leaves (control), the known peptides "PEP4" and "PEP4-rink", the peptide of the invention having SEQ. ID. No. 1 and the native peptide trichogin. The average area of the lesion was obtained by performing at least three biological replicates and was calculated after 7 days by Assess^{©} Software (APS). The treatment with the peptide of the invention decreases the infected area of the treated barley leaves by at least 95% compared to control leaves.

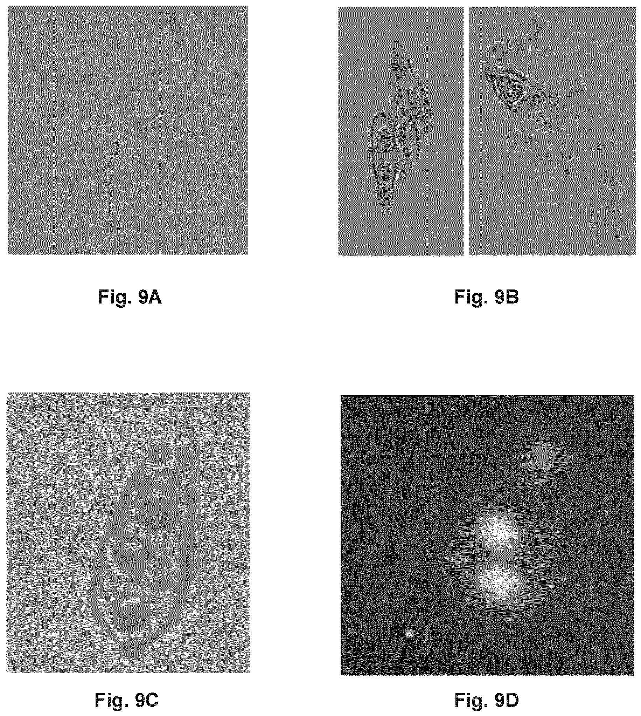

- Figures 9A, 9B, 9C and 9D show optical (Figures 9A, 9B) and fluorescence (Figures 9C, 9D) microscope photographs of untreated tricellular spores of *Pyricularia oryzae* or treated with the peptide of the invention having SEQ. ID. No. 1 at a concentration of 50 µM. Figure 9A shows the untreated spore after 48 hours of incubation in PDB culture medium. The spore germinated normally. Figures 9B, 9C show the spore treated with the peptide having SEQ. ID. No. 1: after 48 hours, germination is inhibited and the cytoplasm is agglutinated. Figure 9D shows the autofluorescence of the agglutinated cytoplasm: autofluorescence indicates that the cells are no longer viable.

### Detailed description of the invention

As mentioned above, the present invention relates to the use of a peptide or of a salt thereof as a plant protection product against pathogenic micro-organisms of the plants, wherein such a peptide has general formula (I)

1-octanoyl-X-Aib-Y-Z (I),

wherein X is selected from the group consisting of Aib-Lys(HCI)-Leu, Aib-Gly-Leu, Leu, or X is absent;
Y is selected from the group consisting of Lys(HCI), Lys(HCl)-Gly-Leu-Aib-Lys(HCI), Lys(HCl)-Lys(HCl)-Leu-Aib-Lys(HCl), Gly-Gly-Leu-Aib-Lys(HCI) and Lys(HCl)-Lys(HCl)-Leu-Aib-Gly;
Z is selected from the group consisting of Lol, Ilol, Ile-NH₂, Leu-NH₂, Ile-Lol and Ile-Leu-NH₂;
and wherein if, at the same time, X is Aib-Gly-Leu or is absent and Y is Lys(HCI)-Lys(HCI)-Leu-Aib-Gly, then Z is not Ile-Lol and is not Ile-Leu-NH₂.

In particular, the use of a peptide having a sequence selected from 1-octanoyl-Aib-Gly-Leu-Aib-Lys(HCl)-Lys(HCl)-Leu-Aib-Gly-Ile-Lol, 1-octanoyl-Aib-Gly-Leu-Aib-Lys(HCl)-Lys(HCl)-Aib-Gly-Ile-Leu-NH₂, 1-octanoyl-Aib-Lys(HCl)-Lys(HCl)-Leu-Aib-Gly-Ile-Lol and 1-octanoyl-Aib-Lys(HCl)-Lys(HCl)-Leu-Aib-Gly-Ile-Leu-NH₂ is to be considered excluded from the present invention.

It should be noted that the peptides having general formula (I) are different from those known, in particular from those described in document WO2020003220, because none of the known peptides have a sequence that is included in the general formula of the present invention and which is to be considered with the exclusions indicated above.

The peptides of the present invention are thus new compared to those described in this document, and, in addition, they show a greater plant protection activity than such known peptides, as will be apparent to a person skilled in the art from the examples given below.

It is specified that, in this document:
- by the term "Lys(HCl)" it is intended the amino acid lysine in its side-chain hydrochloride form; however, it is not excluded that the aforesaid amino acid can be replaced by the amino acid lysine;
- by "1-octanoyl" it is intended as comprising the acyl group derived from the octanoic acid and bound to the N-terminus end of the peptide;
- by "Aib" it is intended the non-coded natural amino acid alpha-aminoisobutyric acid;
- by "Lol" it is intended the 1,2-aminoalcohol L-leucinol;
- and by "Ilol" it is intended the isoleucinol.

With regard to the other amino acids described, they are indicated by their three-letter code: Leu is leucine, Gly is glycine, Ile is isoleucine.

Preferably, the chiral amino acids are in L configuration, unless otherwise specified.

It is further specified that the peptide of the invention may be used alone or in combination with other peptides of the present invention and, in addition, may be prepared in the form of a composition, as will be set forth in more detail later.

According to one aspect of the invention, the aforesaid peptide is used to fight and/or prevent infections caused by oomycetes, fungi and phytopathogenic bacteria in plants.

In particular, the aforesaid peptide is used to fight and/or prevent infections in plants caused by *Botrytis cinerea*, *Plasmopara viticola, Fusarium graminearum, Pyricularia oryzae* and *Xanthomonas campestris.*

Preferably, such a peptide is used to fight and/or prevent infections caused by phytopathogenic bacteria, preferably Gram negative bacteria, more preferably *Xanthomonas campestris.*

More preferably, the peptide is used to fight and/or prevent infections caused by pathogenic micro-organisms on vine plants, cereals and horticultural crops. According to one aspect of the invention, the use of such a peptide provides for dissolving the aforesaid peptide in an aqueous solvent prior to application on the plant(s) to be treated so as to favour the dispersion thereof on the same plant(s).

Preferably, the aforesaid aqueous solvent is water.

Advantageously, in fact, the peptide of the invention is soluble in water at a concentration >10mM.

It should be noted that this peptide is also soluble in polar or protic organic solvents.

Still preferably, the peptide of the invention is applied at a concentration comprised between 10-100 µM on each of the aforesaid plants, more preferably at a concentration of about 50 µM.

However, it is not excluded that the peptide is used and applied on plants at a concentration other than that indicated.

In such a case, an expert in the field is to be considered capable of assessing, depending on the type of plant disease and the severity thereof, what will be the optimal concentration of the peptide for its use according to the invention and the frequency of application thereof on the plants to be treated.

Advantageously, the peptide of the invention applied on the plant shows excellent efficiency in preventing infection by blocking the growth of the pathogenic micro-organism.

These characteristics make its use on plants, especially on crops, very advantageous, especially for the preventive use on plants, especially on vine plants, cereals and horticultural crops.

Without wishing to be bound by any theory, from the results of the experiments carried out, the inventors believe that at least some of the efficacy of the peptide of the invention is due to its ability to cause damages to the cell membranes of the pathogenic micro-organism, inducing cell death. Advantageously, moreover, the aforesaid peptide is a product that is fully degradable into non-toxic amino acids.

The peptide of the invention was in fact found to be completely degraded into amino acids by the action of the enzyme trypsin.

Still advantageously, the peptide of the invention is water-soluble, making its use particularly practical in the field.

Another advantage of using the peptide as a plant protection product is that it is completely harmless to plants at even very high concentrations (1 mM). Preferably, the peptide of the invention is selected from:
1-octanoyl-Aib-Lys(HCl)-Leu-Aib-Lys(HCl)-Gly-Leu-Aib-Lys(HCl)-Ile-Lol (SEQ.ID.No.1 or "K259G6");
1-octanoyl-Aib-Lys(HCl)-Leu-Aib-Lys(HCl)-Gly-Leu-Aib-Lys(HCl)-Ile-Leu-NH₂ (SEQ.ID.No.2 or "K259G6-NH₂");
1-octanoyl-Aib-Lys(HCl)-Leu-Aib-Lys(HCl)-Lys(HCl)-Leu-Aib-Lys(HCl)-Ile-Lol (SEQ.ID.No.3 or "K2569"); 
1-octanoyl-Aib-Lys(HCl)-Leu-Aib-Lys(HCl)-Lys(HCl)-Leu-Aib-Lys(HCl)-Ile-Leu-
NH2 (SEQ.ID.No.4 or “K2569-NH2”);
1-octanoyl-Aib-Gly-Leu-Aib-Gly-Gly-Leu-Aib-Lys(HCl)-Ile-Lol (SEQ.ID.No.5 or "K9");
1-octanoyl-Aib-Gly-Leu-Aib-Gly-Gly-Leu-Aib-Lys(HCl)-Ile-Leu-NH₂ (SEQ.ID.No.6 or "K9-NH₂");
1-octanoyl-Aib-Lys(HCl)-Leu-Aib-Lys(HCl)-Lys(HCl)-Leu-Aib-Gly-Ile-Lol (SEQ.ID.No.7 or "K256");
1-octanoyl-Aib-Lys(HCl)-Leu-Aib-Lys(HCl)-Lys(HCl)-Leu-Aib-Gly-Ile-Leu-NH₂ (SEQ.ID.No.8 or "K256-NH₂");
1-octanoyl-Leu-Aib-Lys(HCl)-Ile-Lol (SEQ.ID.No.9);
1 -octanoyl-Leu-Aib-Lys(HCl)-Ile-Leu-NH₂ (SEQ.ID.No. 10);
1-octanoyl-Leu-Aib-Lys(HCl)-Lol(SEQ.ID.No.11);
1 -octanoyl-Leu-Aib-Lys(HCl)-Leu-NH₂ (SEQ.ID.No. 12);
1-octanoyl-Leu-Aib-Lys(HCl)-Ilol (SEQ.ID.No.13);
1-octanoyl-Leu-Aib-Lys(HCl)-Ile-NH₂ (SEQ.ID.No.14);
1-octanoyl-Aib-Lys(HCl)-Ile-Lol (SEQ.ID.No.15);
1-octanoyl-Aib-Lys(HCl)-Ile-Leu-NH₂ (SEQ.ID.No.16).

According to another aspect of the invention, the peptide has general formula (I) wherein X is selected from Aib-Lys(HCl)-Leu and Aib-Gly-Leu; Y is selected from Lys(HCI)-Gly-Leu-Aib-Lys(HCI), Lys(HCl)-Lys(HCl)-Leu-Aib-Lys(HCl), Gly-Gly-Leu-Aib-Lys(HCI) and Lys(HCl)-Lys(HCl)-Leu-Aib-Gly;Z is selected from Ile-Lol and Ile-Leu-NH₂.

Preferably, according to this aspect of the invention, such a peptide is selected from the group consisting of the peptides having SEQ. ID. Nos. from 1 to 8. Even more preferably, the peptide is selected from the peptides having SEQ. ID. Nos. 2, 4, 6 and 8.

According to a further aspect of the invention, the peptide has general formula (I) wherein X is Leu or X is absent; Y is Lys(HCI) and wherein Z is selected from Lol, Ilol, Ile-NH₂, Leu-NH₂, Ile-Lol and Ile-Leu-NH₂.

In particular, according to this further aspect of the invention, the peptide preferably has a sequence selected from the group consisting of SEQ. ID. Nos. from 9 to 16.

Advantageously, the peptides having SEQ. ID. Nos. from 9 to 16 are particularly suitable for being synthesised by synthetic strategies in solution which, as is known, represent techniques that are easier to carry out and cheaper than solid-phase peptide synthesis.

It is not excluded that, according to embodiment variants, one or more of the peptides of the present invention are made by solid-phase techniques or by techniques other than that indicated.

Therefore, an object of the present invention is also a peptide having general formula (I)

1-octanoyl-X-Aib-Y-Z (I),

wherein X is selected from the group consisting of Aib-Lys(HCI)-Leu, Aib-Gly-Leu, Leu, or X is absent;
Y is selected from the group consisting of Lys(HCI), Lys(HCl)-Gly-Leu-Aib-Lys(HCI), Lys(HCl)-Lys(HCl)-Leu-Aib-Lys(HCl), Gly-Gly-Leu-Aib-Lys(HCI) and Lys(HCl)-Lys(HCl)-Leu-Aib-Gly;
Z is selected from the group consisting of Lol, Ilol, Ile-NH₂, Leu-NH₂, Ile-Lol and Ile-Leu-N H₂;
and wherein if, at the same time, X is Aib-Gly-Leu or is absent and Y is Lys(HCI)-Lys(HCI)-Leu-Aib-Gly, then Z is not Ile-Lol and is not Ile-Leu-NH₂.

In particular, the peptide having a sequence selected from 1-octanoyl-Aib-Gly-Leu-Aib-Lys(HCl)-Lys(HCl)-Leu-Aib-Gly-Ile-Lol, 1-octanoyl-Aib-Gly-Leu-Aib-Lys(HCl)-Lys(HCl)-Aib-Gly-Ile-Leu-NH₂, 1-octanoyl-Aib-Lys(HCl)-Lys(HCl)-Leu-Aib-Gly-Ile-Lol and 1-octanoyl-Aib-Lys(HCl)-Lys(HCl)-Leu-Aib-Gly-Ile-Leu-NH₂ is to be considered excluded from the present invention.

Preferably, the peptide according to the invention has general formula (I) having a sequence selected from the group consisting of SEQ. ID. Nos. from 1 to 16.

According to one aspect of the present invention, the aforesaid peptide has general formula (I) wherein X is selected from Aib-Lys(HCl)-Leu and Aib-Gly-Leu; Y is selected from Lys(HCI)-Gly-Leu-Aib-Lys(HCI), Lys(HCl)-Lys(HCl)-Leu-Aib-Lys(HCI), Gly-Gly-Leu-Aib-Lys(HCI) and Lys(HCl)-Lys(HCl)-Leu-Aib-Gly; Z is selected from Ile-Lol and Ile-Leu-NH₂.

According to this aspect of the invention, the peptide is selected from the peptides having SEQ. ID. Nos. from 1 to 8.

Preferably, the aforesaid peptide is selected from the group consisting of the peptides having SEQ. ID. Nos. 2, 4, 6 and 8.

According to another aspect of the invention, the peptide of the invention has general formula (I) wherein X is Leu or X is absent; Y is Lys(HCI) and Z is selected from Lol, Ilol, Ile-NH₂, Leu-NH₂, Ile-Lol and Ile-Leu-NH₂.

Preferably, this peptide is selected from the peptides having SEQ. ID. Nos. from 9 to 16.

More preferably, such a peptide is the peptide having SEQ. ID. No. 10. According to one aspect of the invention, the peptide of the present invention is 1-octanoyl-Aib-Lys(HCl)-Leu-Aib-Lys(HCl)-Gly-Leu-Aib-Lys(HCl)-Ile-Lol (SEQ.ID.No.1 or "K259G6").

According to one aspect of the invention, the peptide of the present invention is 1-octanoyl-Aib-Lys(HCl)-Leu-Aib-Lys(HCl)-Gly-Leu-Aib-Lys(HCl)-Ile-Leu-NH₂ (SEQ.ID.No.2 or "K259G6-NH₂").

According to one aspect of the invention, the peptide of the present invention is 1-octanoyl-Aib-Lys(HCl)-Leu-Aib-Lys(HCl)-Lys(HCl)-Leu-Aib-Lys(HCl)-Ile-Lol (SEQ.ID.No.3 or "K2569").

According to one aspect of the invention, the peptide of the present invention is 1-octanoyl-Aib-Lys(HCl)-Leu-Aib-Lys(HCl)-Lys(HCl)-Leu-Aib-Lys(HCl)-Ile-Leu-NH₂ (SEQ.ID.No.4 or "K2569-NH₂").

According to one aspect of the invention, the peptide of the present invention is 1-octanoyl-Aib-Gly-Leu-Aib-Gly-Gly-Leu-Aib-Lys(HCl)-Ile-Lol (SEQ.ID.No.5 or "K9").

According to one aspect of the invention, the peptide of the present invention is 1-octanoyl-Aib-Gly-Leu-Aib-Gly-Gly-Leu-Aib-Lys(HCl)-Ile-Leu-NH₂ (SEQ.ID.No.6 or "K9-NH₂").

According to one aspect of the invention, the peptide of the present invention is 1-octanoyl-Aib-Lys(HCl)-Leu-Aib-Lys(HCl)-Lys(HCl)-Leu-Aib-Gly-Ile-Lol (SEQ.ID.No.7 or "K256").

According to one aspect of the invention, the peptide of the present invention is 1-octanoyl-Aib-Lys(HCl)-Leu-Aib-Lys(HCl)-Lys(HCl)-Leu-Aib-Gly-Ile-Leu-NH₂ (SEQ.ID.No.8 or "K256-NH₂").

According to one aspect of the invention, the peptide of the present invention is 1-octanoyl-Leu-Aib-Lys(HCl)-Ile-Lol (SEQ.ID.No.9).

According to one aspect of the invention, the peptide of the present invention is 1-octanoyl-Leu-Aib-Lys(HCl)-Ile-Leu-NH₂ (SEQ.ID.No.10).

According to one aspect of the invention, the peptide of the present invention is 1-octanoyl-Leu-Aib-Lys(HCl)-Lol (SEQ.ID.No.11).

According to one aspect of the invention, the peptide of the present invention is 1-octanoyl-Leu-Aib-Lys(HCl)-Leu-NH₂ (SEQ.ID.No.12).

According to one aspect of the invention, the peptide of the present invention is 1-octanoyl-Leu-Aib-Lys(HCl)-Ilol (SEQ.ID.No.13).

According to one aspect of the invention, the peptide of the present invention is 1-octanoyl-Leu-Aib-Lys(HCl)-Ile-NH₂ (SEQ.ID.No.14).

According to one aspect of the invention, the peptide of the present invention is 1-octanoyl-Aib-Lys(HCl)-Ile-Lol (SEQ.ID.No.15).

According to one aspect of the invention, the peptide of the present invention is 1-octanoyl-Aib-Lys(HCl)-Ile-Leu-NH₂ (SEQ.ID.No.16).

As indicated above, the present invention also relates to a plant protection composition comprising at least one peptide of the invention, or a salt thereof, as defined above, including variants, and a phytopharmaceutically acceptable carrier and/or excipient.

Preferably, the aforesaid carrier is water.

According to the invention, the plant protection composition comprises at least one peptide of the invention, or a salt thereof, selected from the peptides having SEQ. ID. Nos. from 1 to 16.

According to one aspect of the plant protection composition of the invention, the plant protection composition comprises at least one peptide of the invention, or a salt thereof, having general formula (I) wherein X is selected from Aib-Lys(HCl)-Leu and Aib-Gly-Leu; Y is selected from Lys(HCl)-Gly-Leu-Aib-Lys(HCI), Lys(HCl)-Lys(HCl)-Leu-Aib-Lys(HCl), Gly-Gly-Leu-Aib-Lys(HCl) and Lys(HCI)-Lys(HCI)-Leu-Aib-Gly, and wherein Z is selected from Ile-Lol and Ile-Leu-N H₂.

Preferably, such at least one peptide is selected from the peptides having SEQ. ID. Nos. from 1 to 8.

More preferably, according to this aspect of the composition of the invention, the aforesaid composition comprises at least one peptide selected from the peptides having SEQ. ID. Nos. 2, 4, 6 and 8.

According to another aspect of the invention, the plant protection composition comprises at least one peptide of the invention, or a salt thereof, having general formula (I) wherein X is Leu or X is absent; Y is Lys(HCI) and wherein Z is selected from Lol, Ilol, Ile-NH₂, Leu-NH₂, Ile-Lol and Ile-Leu-NH₂. Preferably, such at least one peptide is selected from the peptides of the invention having SEQ. ID. Nos. from 9 to 16.

It should be noted that the plant protection composition of the invention may comprise one or more peptides of the present invention described above. Additionally, the plant protection composition of the invention may further comprise one or more excipients of the known type which will be selected according to usual practice.

Such excipients may include, among others, co-formulant compounds that serve to reduce the concentration of the peptide, such as inert substances and diluents.

In addition, these excipients may include adjuvant compounds, which are intended to increase the efficacy of the peptide and promote its distribution. Examples of such adjuvants comprise synergists, emulsifiers, wetting agents, adhesives, humectants, propellants for aerosol, inert diluents, anti-drift, antifoaming, preservative formulations, and the like.

The plant protection composition of the present invention may be in liquid or solid form.

Examples of liquid formulations comprise aqueous solutions in which the peptide is finely dissolved in water to form a stable, diluted or concentrated solution.

Optionally, this solution is also mixed with wetting, dispersing, inert and other per se known excipients.

The formulation of the plant protection composition of the invention in liquid form is particularly preferred for ease of application on the plant by spraying. Examples of solid formulations of the plant protection composition of the invention comprise granules and dry powders.

Although the peptide of the invention is already itself absorbable by the plant on which it is applied, it is not excluded that the plant protection composition of the invention comprising this peptide is used in the form of a formulation for endotherapic treatments, i.e. by injection of the same into the trunk of the plant.

In such endotherapic formulation, the plant protection composition will comprise adjuvants especially formulated and known in the state of the art for being injected along the xylematic vessels and spreading along them.

The plant protection composition of the invention is advantageously suitable for use as a plant protection product in plants.

In particular, the aforesaid composition is particularly suitable for use for the treatment of vine plants, cereals and horticultural crops, preferably for the treatment and/or prevention of plants against infections caused by *Botrytis cinerea*, *Plasmopara viticola*, *Fusarium graminearum*, *Pyricularia oryzae* or *Xanthomonas campestris.*

The use of the previously defined plant protection composition, including variants, as a plant protection product against pathogenic micro-organisms of plants, preferably as a plant protection product for the treatment and/or prevention of infections in plants caused by oomycetes, fungi and phytopathogenic bacteria of plants, such plants being preferably vine plants, cereals, horticultural crops is therefore also part of the present invention. Further characteristics and advantages of the peptides, the plant protection composition and their use will be apparent to a person skilled in the art from the examples below, which are provided for the purpose of a better understanding of what is described and are not intended as limitations of the claims.

### EXAMPLES

### Example 1. Synthesis of the peptides in solution and purification/ characterisation of the peptides obtained

The peptide of the present invention can be synthesised following a manual, semi-automatic or automatic solid phase, solution synthesis methodology. The chiral amino acids used have an L configuration, unless otherwise specified. Synthesis in solution takes place from the primary amide of the last amino acid of the desired sequence.

An exemplary synthetic protocol in solution starts from the synthesis of H-Leu-NH₂, from H-Leu-OCH₃ (leucine methyl ester) and ammonia in methanol. The synthesis proceeds with the condensation, using appropriate activating reagents, of the subsequent amino acid, protected to the amino function, e.g. as benzyloxycarbonyl (Z). The removal of the protective group (e.g. by catalytic hydrogenation) closes the cycle. The subsequent residues are incorporated by a similar process. Each intermediate is isolated and characterised. The amino acid lysine is inserted protected in side chain (e.g. as *tert*-butoxycarbonyl). This protective group is removed as the last stage of the synthesis, by acid treatment (e.g. with a mixture of trifluoroacetic acid in dichloromethane).

The raw peptides are obtained with a degree of purity greater than 70% and purified at >95% by chromatography, flash chromatography on silica, semi-automatic medium pressure chromatography or preparative high pressure liquid chromatography (HPLC).

The characterisation of the peptides and the determination of the degree of purity are obtained by high-resolution mass spectrometry analysis *(electron spray ionization time-of-fly, ESI-TOF)*, analytical HPLC, nuclear magnetic resonance (NMR).

### Example 2. Chemoenzymatic peptide synthesis and purification/ characterisation of the peptides obtained

For the chemoenzymatic peptide synthesis, protected residues and/or segments of the sequence are synthesised in solution which are then condensed by means of enzymes such as the enzyme papain (Kayo T. et al, ACS Biomaterials Science & Engineering, 2020), trypsin or others. Chemoenzymatic synthesis, for example, provides for the preparation of protected residues such as Z and benzyl esters or as amides. Each residue may have one or more enzymes that catalyse the formation of the peptide bond.

### Example 3. Solid phase peptide synthesis and purification/characterisation of the peptides obtained

An example of a synthetic solid-phase protocol uses "amide Rink" resins or 2-chlorotritic resin preloaded with aminoalcohol L-leucinol or Isoleucinol. Both are commercial resins and used at 100-200 or 200-400 mesh and different loading degree.

The synthesis proceeds starting from the C-terminus amino acid towards the N-terminus amino acid with a step-by-step process.

The protocol provides for the use of the fluorenylmethyloxycarbonyl- (Fmoc-) protective group for the protection of the amino group in alpha. This protective group is removed in basic conditions, for example by treatment with piperidine (PIP) 20% in dimethylformamide (DMF) or other secondary amines in different percentages and in other organic solvents. The protective group used for the protection of the amino group in the side chain is *tert*-butyloxycarbonyl (Boc), removable by acid treatment, for example with a solution of HCI 3M in methanol or other organic solvents, or trifluoroacetic acid in different percentages in dichloromethane or other organic solvents.

The formation reactions of the amide bond take place through activation of the carboxyl group of the incoming amino acid (protected in its amine function and possible side chains) through the use of different activating agents depending on the amino acid.

By way of non-limiting example, it is possible to use: (i) a 1:1 mixture of 1-hydroxy-1,2,3-benzotriazole (HOBt) and O-(benzotriazol-1-yl)-1,1,3,3-tetramethyluronium hexafluoro phosphate (HBTU); (ii) a 1:1 mixture of 1-hydroxy-1,2,3-benzotriazole (HOBt) and O-(benzotriazol-1-yl)-1,1,3,3-tetramethyluronium tetrafluoroborate (TBTU); (iii) O-(7-azabenzotriazol-1-yl) - 1,1,3,3-tetramethyluronium hexafluorophosphate (HATU); (iv) ethyl cyano(hydroxyimino)acetate (Oxyma pure); (v) K-Oxyma; (vi) a 1:1 mixture of *N*-ethyl-*N'*-(3-dimethylamino)propylcarbodiimide (EDC) or diisopropylcarbodiimide (DIC) or dicyclohexylcarbodiimide (DCC) and HOBt; (vii) a 1:1 mixture of EDC or DIC or DCC and HOAt; again in DMF solution. The incoming amino acid excess varies between 1.5 and 3 equivalents and the activating reactants are added in an equimolar quantity with respect to the incoming amino acid. The tertiary base is added in twice the amount of the incoming amino acid when HATU or HBTU are agents used as activators. Base addition is not necessary with OXYMA PURE or K-Oxyma.

As a tertiary base it can used (by way of non-limiting example): diisopropylethylamine, triethylamine, or N-methylmorpholine.

The n-octanoyl group is inserted at the N-terminus end using the same methods of activation of the carboxylic group previously described. The formation reactions of the amide bond also take place through the use of the enzyme papaine. The peptide is released from the resin, previously dried with dichloromethane washes and permanence under vacuum, by acid treatment. By way of example: from the "amide Rink" resin, the peptide can be released by treatment with a mixture of trifluoroacetic acid (TFA) 95%, water 2.5% and triisopropylsilane 2.5%. The collected solution is brought to dryness. The solid or oily precipitate obtained is then washed several times with diethyl ether; from the 2-chlorotritilic resin, the peptide is released with repeated treatments of variable duration from one hour to overnight with a solution of 1,1,1,3,3,3-hexafluoroisopropanol (HFIP) 30% in dichloromethane (DCM). Alternatively, a solution with various percentages of TFA in DCM can be also used. When using the 2-chlorotryl resin, the protective group Boc is removed in solution by the treatments described above, e.g. by treatment with 3M hydrochloric acid in methanol. In some cases, the protected peptides Boc are purified by aqueous acid and/or basic washes, followed by anhydrification over anhydrous Na₂SO₄ or MgSO₄.

The raw peptides are obtained with a degree of purity greater than 70% and purified at >95% by chromatography, flash chromatography on silica, semi-automatic medium pressure chromatography or preparative high pressure liquid chromatography (HPLC).

The characterisation of the peptides and the determination of the degree of purity are obtained by high-resolution mass spectrometry analysis *(electron spray ionization time-of-fly, ESI-TOF)*, analytical HPLC, nuclear magnetic resonance (NMR).

### Example 4. Production of the spores of the phytopathogenic fungi

*Botrytis cinerea* (strain PM10) was cultivated at 25°C on potato dextrose agar (PDA). *Pyricularia oryzae* (strain IT10) was initially cultivated on PDA culture medium at 25°C and then on *oat meal agar* (OMA). *F. graminearum* (strain 8/1) was cultivated on PDA at 25°C and subsequently on synthetic nutrient agar (SNA). After a few days the spores (conidia) of each plate were collected in 6 mL of sterile water containing glycerol (10% *v*/*v*).

### Example 5. In vitro activity of the peptides against phytopathogenic fungi

The antifungal activity of the peptides was determined against *B*. *cinerea, P. oryzae* and *F. graminearum* in microtitre plates containing in each well: 200 µL of potato dextrose broth (PDB; pH 6.9), the peptide of interest and, alternatively to each other, spores of *B*. *cinerea* (5 × 10⁵ mL⁻¹), *F. graminearum* (5×10⁵ mL⁻¹) or *P. oryzae* (1×10⁵ mL⁻¹). Peptide-free controls were also prepared. Each peptide was tested in three independent replicates.

After 96 hours of incubation in the dark at 25°C, growth was measured spectrophotometrically at 450 nm and fungal growth was expressed as a percentage of the absorbance values of each well compared to the maximum absorbance measured in the control wells not inoculated with peptides.

### Example 6. In vitro activity against Xanthomonas campestris pv. campestris

The peptides were assayed in microtitre plates against a strain of X. *campestris* pv. *campestris* isolated from cauliflower. Each well contained 200 µL of LB substrate, 1×10⁶ CFU/mL of bacterium and the peptide of interest (15 µM). The reduction in growth was determined after 48h by spectrophotometrically measuring the reduction in turbidity (A₆₀₀ nm) of the wells containing the peptides compared to the wells without peptides. Each peptide was assayed in 3 different wells.

### Example 7. Microscopic analysis of the fungi

Microscopic observations of the peptide-treated spores were performed by optical microscopy (Laborlux 12) after 24 hours suspension of the spores in PDB. The spores of *P. oryzae* were also observed up to 48h by fluorescence microscopy (Leica DM 4000B).

### Example 8. Peptide treatments and experiments of infection caused by P. viticola

Infection tests were performed on vine leaf disks (cv. Glera) of 1 cm in diameter. Twenty leaf discs obtained from randomly collected leaves from different plants were placed with the lower side facing upwards on moistened sterile paper placed in Petri dishes (15 cm diameter). Approximately 0.1 mL of each aqueous solution containing the peptide (50 µM) was distributed on each leaf disc. Aqueous suspensions of *P. viticola* sporangia, collected from infected vine plants, were diluted to obtain 3-5×10⁵ sporangia/mL and distributed on the leaf discs. The plates containing the leaf discs were incubated in the dark at room temperature (22-23°C). The incidence was calculated 12 days after inoculation as the ratio of the number of sporulating discs to the total number of discs inoculated. Control plates contained water-treated leaf discs. Two or three plates were used for each treatment. The data obtained were statistically analysed by applying the Anova and Bonferroni-Holmes one-way test.

### Example 9. Peptide treatments and experiments of infection on barley leaves with P. oryzae

The first leaves of 6-day-old barley seedlings were inoculated at two points by pipetting 10 µL of a suspension containing the peptide at 50 µM and 1×10³ spores of *P. oryzae* strain IT10. For each peptide, at least five leaves were inoculated in each experiment and the experiment was performed at least three times. Water-treated leaves inoculated with fungal conidia were used as a positive control. The lesion area was calculated after 7 days by Assess^{©} Software (APS).

### Example 10. Peptide treatments and experiments of infection on ears of wheat with F. graminearum

Flowering ears of wheat were inoculated by spraying with a solution containing 50 µM of each peptide and 5×10⁵ spores mL⁻¹ of *F. graminearum.* At least eight ears were inoculated for each treatment in each experiment. Ears sprayed with water and fungal spores were used as a positive control. The percentage of infection was determined by counting the number of visually symptomatic spikelets compared to the total number of spikelets of the respective ear. The experiments with each peptide were repeated at least three times.

## Claims

1. Use of a peptide or of a salt thereof as a plant protection product against pathogenic micro-organisms of the plants, said peptide having the following general formula (I)
1-octanoyl-X-Aib-Y-Z (I),
wherein X is selected from the group consisting of Aib-Lys(HCI)-Leu, Aib-Gly-Leu, Leu, or X is absent;
Y is selected from the group consisting of Lys(HCI), Lys(HCl)-Gly-Leu-Aib-Lys(HCI), Lys(HCl)-Lys(HCl)-Leu-Aib-Lys(HCl), Gly-Gly-Leu-Aib-Lys(HCl) and Lys(HCl)-Lys(HCl)-Leu-Aib-Gly;
Z is selected from the group consisting of Lol, Ilol, Ile-NH₂, Leu-NH₂, Ile-Lol and Ile-Leu-N H₂;
and wherein if, at the same time, X is Aib-Gly-Leu or is absent and Y is Lys(HCI)-Lys(HCI)-Leu-Aib-Gly, then Z is not Ile-Lol and is not Ile-Leu-NH₂.

2. The use according to the preceding claim, **characterised in that** said peptide is used to fight and/or prevent infections in plants caused by oomycetes, fungi and phytopathogenic bacteria.

3. The use according to any one of the preceding claims, **characterised in that** said peptide is used to fight and/or prevent infections in plants caused by *Botrytis cinerea*, *Plasmopara viticola*, *Fusarium graminearum*, *Pyricularia oryzae* and *Xanthomonas campestris*, preferably *Xanthomonas campestris.*

4. The use according to any one of the preceding claims, **characterised in that** said peptide is dissolved in an aqueous solvent prior to application on said plants, said peptide being preferably applied at a concentration comprised between 10-100 µM for each of said plants, preferably at a concentration of about 50 µM.

5. The use according to any one of the preceding claims, **characterised in that** X is selected from Aib-Lys(HCl)-Leu and Aib-Gly-Leu; Y is selected from Lys(HCI)-Gly-Leu-Aib-Lys(HCI), Lys(HCl)-Lys(HCl)-Leu-Aib-Lys(HCl), Gly-Gly-Leu-Aib-Lys(HCI) and Lys(HCl)-Lys(HCl)-Leu-Aib-Gly; Z is selected from Ile-Lol and Ile-Leu-NH₂.

6. The use according to the preceding claim, **characterised in that** said peptide is selected from:
1-octanoyl-Aib-Lys(HCl)-Leu-Aib-Lys(HCl)-Gly-Leu-Aib-Lys(HCl)-Ile-Lol (SEQ.ID.No.1 or "K259G6");
1-octanoyl-Aib-Lys(HCl)-Leu-Aib-Lys(HCl)-Gly-Leu-Aib-Lys(HCl)-Ile-Leu-NH₂ (SEQ.ID.No.2 or "K259G6-NH₂");
1-octanoyl-Aib-Lys(HCl)-Leu-Aib-Lys(HCl)-Lys(HCl)-Leu-Aib-Lys(HCl)-Ile-Lol (SEQ.ID.No.3or "K2569"); 
1-octanoyl-Aib-Lys(HCl)-Leu-Aib-Lys(HCl)-Lys(HCl)-Leu-Aib-Lys(HCl)-Ile-Leu-
NH2 (SEQ.ID.No.4 or “K2569-NH2”);
1-octanoyl-Aib-Gly-Leu-Aib-Gly-Gly-Leu-Aib-Lys(HCl)-Ile-Lol (SEQ.ID.No.5 or "K9");
1-octanoyl-Aib-Gly-Leu-Aib-Gly-Gly-Leu-Aib-Lys(HCl)-Ile-Leu-NH₂ (SEQ.ID.No.6 or "K9-NH₂");
1-octanoyl-Aib-Lys(HCl)-Leu-Aib-Lys(HCl)-Lys(HCl)-Leu-Aib-Gly-Ile-Lol (SEQ.ID.No.7 or "K256");
1-octanoyl-Aib-Lys(HCl)-Leu-Aib-Lys(HCl)-Lys(HCl)-Leu-Aib-Gly-Ile-Leu-NH₂ (SEQ.ID.No.8 or "K256-NH₂").

7. The use according to the preceding claim, **characterised in that** said peptide is selected from:
1-octanoyl-Aib-Lys(HCl)-Leu-Aib-Lys(HCl)-Gly-Leu-Aib-Lys(HCl)-Ile-Leu-NH₂ (SEQ.ID.No.2 or "K259G6-NH₂"); 
1-octanoyl-Aib-Lys(HCl)-Leu-Aib-Lys(HCl)-Lys(HCl)-Leu-Aib-Lys(HCl)-Ile-Leu-
NH2 (SEQ.ID.No.4 or “K2569-NH2”);
1-octanoyl-Aib-Gly-Leu-Aib-Gly-Gly-Leu-Aib-Lys(HCl)-Ile-Leu-NH₂ (SEQ.ID.No.6 or "K9-NH₂");
1-octanoyl-Aib-Lys(HCl)-Leu-Aib-Lys(HCl)-Lys(HCl)-Leu-Aib-Gly-Ile-Leu-NH₂ (SEQ.ID.No.8 or "K256-NH₂").

8. The use according to any one of claims 1 to 4, **characterised in that** X is Leu or X is absent; Y is Lys(HCl); Z is selected from Lol, Ilol, Ile-NH₂, Leu-NH₂, Ile-Lol and Ile-Leu-NH₂.

9. The use according to the preceding claim, **characterised in that** said peptide is selected from:
1-octanoyl-Leu-Aib-Lys(HCl)-Ile-Lol (SEQ.ID.No.9);
1-octanoyl-Leu-Aib-Lys(HCl)-Ile-Leu-NH₂ (SEQ.ID.No.10);
1-octanoyl-Leu-Aib-Lys(HCl)-Lol(SEQ.ID.No.11);
1-octanoyl-Leu-Aib-Lys(HCl)-Leu-NH₂ (SEQ.ID.No.12);
1-octanoyl-Leu-Aib-Lys(HCl)-Ilol (SEQ.ID.No.13);
1-octanoyl-Leu-Aib-Lys(HCl)-Ile-NH₂ (SEQ.ID.No.14);
1-octanoyl-Aib-Lys(HCl)-Ile-Lol (SEQ.ID.No.15);
1-octanoyl-Aib-Lys(HCl)-Ile-Leu-NH₂ (SEQ.ID.No.16).

10. The use according to any one of claims 1 to 4, **characterised in that** said peptide is selected from:
1-octanoyl-Aib-Lys(HCl)-Leu-Aib-Lys(HCl)-Gly-Leu-Aib-Lys(HCl)-Ile-Lol (SEQ.ID.No.1 or "K259G6");
1-octanoyl-Aib-Lys(HCl)-Leu-Aib-Lys(HCl)-Gly-Leu-Aib-Lys(HCl)-Ile-Leu-NH₂ (SEQ.ID.No.2 or "K259G6-NH₂");
1-octanoyl-Aib-Lys(HCl)-Leu-Aib-Lys(HCl)-Lys(HCl)-Leu-Aib-Lys(HCl)-Ile-Lol (SEQ.ID.No.3or "K2569"); 
1-octanoyl-Aib-Lys(HCl)-Leu-Aib-Lys(HCl)-Lys(HCl)-Leu-Aib-Lys(HCl)-Ile-Leu-
NH2 (SEQ.ID.No.4 or “K2569-NH2”);
1-octanoyl-Aib-Gly-Leu-Aib-Gly-Gly-Leu-Aib-Lys(HCl)-Ile-Lol (SEQ.ID.No.5 or "K9");
1-octanoyl-Aib-Gly-Leu-Aib-Gly-Gly-Leu-Aib-Lys(HCl)-Ile-Leu-NH₂ (SEQ.ID.No.6 or "K9-NH₂");
1-octanoyl-Aib-Lys(HCl)-Leu-Aib-Lys(HCl)-Lys(HCl)-Leu-Aib-Gly-Ile-Lol (SEQ.ID.No.7 or "K256");
1-octanoyl-Aib-Lys(HCl)-Leu-Aib-Lys(HCl)-Lys(HCl)-Leu-Aib-Gly-Ile-Leu-NH₂ (SEQ.ID.No.8 or "K256-NH₂");
1-octanoyl-Leu-Aib-Lys(HCl)-Ile-Lol (SEQ.ID.No.9);
1-octanoyl-Leu-Aib-Lys(HCl)-Ile-Leu-NH₂ (SEQ.ID.No.10);
1-octanoyl-Leu-Aib-Lys(HCl)-Lol(SEQ.ID.No.11);
1-octanoyl-Leu-Aib-Lys(HCl)-Leu-NH₂ (SEQ.ID.No.12);
1-octanoyl-Leu-Aib-Lys(HCl)-Ilol (SEQ.ID.No.13);
1-octanoyl-Leu-Aib-Lys(HCl)-Ile-NH₂ (SEQ.ID.No.14);
1-octanoyl-Aib-Lys(HCl)-Ile-Lol (SEQ.ID.No.15);
1-octanoyl-Aib-Lys(HCl)-Ile-Leu-NH₂ (SEQ.ID.No.16).

11. Plant protection composition comprising at least one peptide or a salt thereof as defined in any one of claims 1 to 10 and a phytopharmaceutically acceptable carrier and/or excipient.

12. Plant protection composition according to the preceding claim, **characterised in that** said peptide is selected from:
1-octanoyl-Aib-Lys(HCl)-Leu-Aib-Lys(HCl)-Gly-Leu-Aib-Lys(HCl)-Ile-Lol (SEQ.ID.No.1 or "K259G6");
1-octanoyl-Aib-Lys(HCl)-Leu-Aib-Lys(HCl)-Gly-Leu-Aib-Lys(HCl)-Ile-Leu-NH₂ (SEQ.ID.No.2 or "K259G6-NH₂");
1-octanoyl-Aib-Lys(HCl)-Leu-Aib-Lys(HCl)-Lys(HCl)-Leu-Aib-Lys(HCl)-Ile-Lol (SEQ.ID.No.3or "K2569"); 
1-octanoyl-Aib-Lys(HCl)-Leu-Aib-Lys(HCl)-Lys(HCl)-Leu-Aib-
Lys(HCl)-Ile-Leu-NH2 (SEQ.ID.No.4 or "K2569-NH2");
1-octanoyl-Aib-Gly-Leu-Aib-Gly-Gly-Leu-Aib-Lys(HCl)-Ile-Lol (SEQ.ID.No.5 or "K9");
1-octanoyl-Aib-Gly-Leu-Aib-Gly-Gly-Leu-Aib-Lys(HCl)-Ile-Leu-NH₂ (SEQ.ID.No.6 or "K9-NH₂");
1-octanoyl-Aib-Lys(HCl)-Leu-Aib-Lys(HCl)-Lys(HCl)-Leu-Aib-Gly-Ile-Lol (SEQ.ID.No.7 or "K256");
1-octanoyl-Aib-Lys(HCl)-Leu-Aib-Lys(HCl)-Lys(HCl)-Leu-Aib-Gly-Ile-Leu-NH₂ (SEQ.ID.No.8 or "K256-NH₂");
1-octanoyl-Leu-Aib-Lys(HCl)-Ile-Lol (SEQ.ID.No.9);
1-octanoyl-Leu-Aib-Lys(HCl)-Ile-Leu-NH₂ (SEQ.ID.No.10);
1-octanoyl-Leu-Aib-Lys(HCl)-Lol(SEQ.ID.No.11);
1-octanoyl-Leu-Aib-Lys(HCl)-Leu-NH₂ (SEQ.ID.No.12);
1-octanoyl-Leu-Aib-Lys(HCl)-Ilol (SEQ.ID.No.13);
1-octanoyl-Leu-Aib-Lys(HCl)-Ile-NH₂ (SEQ.ID.No.14);
1-octanoyl-Aib-Lys(HCl)-Ile-Lol (SEQ.ID.No.15);
1-octanoyl-Aib-Lys(HCl)-Ile-Leu-NH₂ (SEQ.ID.No.16).

13. Use of a plant protection composition as defined in any one of claims 11 or 12, as a plant protection product against pathogenic micro-organisms of the plants, preferably as a plant protection product for the treatment and/or prevention of infections in plants caused by fungi, oomycetes and phytopathogenic bacteria, said plants being preferably vine plants, cereal and horticultural crops.

14. A peptide having general formula (I)
1-octanoyl-X-Aib-Y-Z (I),
wherein X is selected from the group consisting of Aib-Lys(HCI)-Leu, Aib-Gly-Leu, Leu, or X is absent;
Y is selected from the group consisting of Lys(HCI), Lys(HCl)-Gly-Leu-Aib-Lys(HCI), Lys(HCl)-Lys(HCl)-Leu-Aib-Lys(HCl), Gly-Gly-Leu-Aib-Lys(HCI) and Lys(HCl)-Lys(HCl)-Leu-Aib-Gly;
Z is selected from the group consisting of Lol, Ilol, Ile-NH₂, Leu-NH₂, Ile-Lol and Ile-Leu-NH₂;
and wherein if, at the same time, X is Aib-Gly-Leu or is absent and Y is Lys(HCI)-Lys(HCI)-Leu-Aib-Gly, then Z is not Ile-Lol and is not Ile-Leu-NH₂.

15. The peptide according to the preceding claim, **characterised in that** it has general formula (I) selected from:
1-octanoyl-Aib-Lys(HCl)-Leu-Aib-Lys(HCl)-Gly-Leu-Aib-Lys(HCl)-Ile-Lol (SEQ.ID.No.1 or "K259G6");
1-octanoyl-Aib-Lys(HCl)-Leu-Aib-Lys(HCl)-Gly-Leu-Aib-Lys(HCl)-Ile-Leu-NH₂ (SEQ.ID.No.2 or "K259G6-NH₂");
1-octanoyl-Aib-Lys(HCl)-Leu-Aib-Lys(HCl)-Lys(HCl)-Leu-Aib-Lys(HCl)-Ile-Lol (SEQ.ID.No.3 or "K2569"); 
1-octanoyl-Aib-Lys(HCl)-Leu-Aib-Lys(HCl)-Lys(HCl)-Leu-Aib-Lys(HCl)-Ile-Leu-
NH2 (SEQ.ID.No.4 or “K2569-NH2”);
1-octanoyl-Aib-Gly-Leu-Aib-Gly-Gly-Leu-Aib-Lys(HCl)-Ile-Lol (SEQ.ID.No.5 or "K9");
1-octanoyl-Aib-Gly-Leu-Aib-Gly-Gly-Leu-Aib-Lys(HCl)-Ile-Leu-NH₂ (SEQ.ID.No.6 or "K9-NH₂");
1-octanoyl-Aib-Lys(HCl)-Leu-Aib-Lys(HCl)-Lys(HCl)-Leu-Aib-Gly-Ile-Lol (SEQ.ID.No.7 or "K256");
1-octanoyl-Aib-Lys(HCl)-Leu-Aib-Lys(HCl)-Lys(HCl)-Leu-Aib-Gly-Ile-Leu-NH₂ (SEQ.ID.No.8 or "K256-NH₂");
1-octanoyl-Leu-Aib-Lys(HCl)-Ile-Lol (SEQ.ID.No.9);
1-octanoyl-Leu-Aib-Lys(HCl)-Ile-Leu-NH₂ (SEQ.ID.No.10);
1-octanoyl-Leu-Aib-Lys(HCl)-Lol(SEQ.ID.No.11);
1-octanoyl-Leu-Aib-Lys(HCl)-Leu-NH₂ (SEQ.ID.No.12);
1-octanoyl-Leu-Aib-Lys(HCl)-Ilol (SEQ.ID.No.13);
1-octanoyl-Leu-Aib-Lys(HCl)-Ile-NH₂ (SEQ.ID.No.14);
1-octanoyl-Aib-Lys(HCl)-Ile-Lol (SEQ.ID.No.15);
1-octanoyl-Aib-Lys(HCl)-Ile-Leu-NH₂ (SEQ.ID.No.16).
